# EUROPÄISCHE PATENTSCHRIFT

(11) **EP 1 493 461 B1**
(45) Veröffentlichungstag und Bekanntmachung des Hinweises auf die Patenterteilung: **04.10.2006**
(21) Anmeldenummer: 04022930.4
(22) Anmeldetag: 12.11.2001
(51) Int. Cl.: A61M 16/06

(54) **Atemmaske mit einer Stirnstütze**
Breathing mask with a forehead support
Masque respiratoire muni d'un support frontal

(30) Priorität: 14.11.2000 DE 10056331
(43) Veröffentlichungstag der Anmeldung: 05.01.2005
(62) Teilanmeldung aus: 01126897.6
(73) Patentinhaber: Weinmann Geräte für Medizin GmbH & Co. KG, 22525 Hamburg (DE)
(72) Erfinder: Eifler, Martin, 25348 Glückstadt (DE); Schulz, Gerd, 22869 Schenefeld (DE)
(74) Vertreter: Klickow, Hans-Henning

(56) Entgegenhaltungen:
- DE-U- 29 923 126
- US-A- 2 245 658
- US-A- 6 119 693

## Beschreibung

Die Erfindung betrifft eine Atemmaske mit einer Vorrichtung zur Abstützung der Atemmaske im Bereich einer Stirn eines Patienten, die mindestens ein im Bereich mindestens eines Halterungsschenkels der Atemmaske angeordnetes und über eine Verstelleinrichtung mit dem Halterungsschenkel verbundenes Stützelement aufweist, wobei das Stützelement ein Stirnpolster trägt.

Derartige Stützelemente mit Verstelleinrichtung dienen dazu, eine an eine jeweilige Gesichtsgeometrie des Patienten angepaßte Abstützung einer Atemmaske im Bereich einer Stirn des Patienten vorzunehmen. Die bekannten Stirnstützen weisen entweder eine Verstellmöglichkeit auf, um eine Verstellung im wesentlichen senkrecht zur Stirn vorzunehmen und hierdurch den wirksamen Stirnabstand zu verändern, oder um eine Verstellung im wesentlichen parallel zur Gesichtsfläche vorzunehmen und hierdurch eine Anpassung an die jeweilige Stirnhöhe durchzuführen. Bekannt sind hierzu mechanische Verstellungen oder der gezielte Einbau von Elementen mit einer relativ zueinander unterschiedlichen geeigneten Dimensionierung.

Eine Stirnstütze mit einer Verstelleinrichtung zur Vorgabe eines wirksamen Stirnabstandes ist beispielsweise in der US-A-61 19 693 beschrieben. Es wird hier eine brückenartige Stirnstütze offenbart, die über zwei Tragarme mit der Atemmaske verbunden ist. Ein Querträger des Stützelementes, der zwei Stirnpolster trägt, weist im Bereich seiner den Stützarmen zugewandten Ausdehnung Rastungen auf. Durch eine Arretierung der Stützarme in unterschiedlichen Rastungen wird ein unterschiedlicher Abstand des Querträgers zur Atemmaske und damit ein unterschiedlich wirksamer Stirnabstand vorgegeben. Das Dokument US-A-61 19 639 zeigt eine Atemmaske gemäß dem Oberbegriff des Patentanspruches 1.

Die bislang bekannten Vorrichtungen können im Hinblick auf ihren Einstellkomfort und ihre Bedienbarkeit noch nicht alle Anforderungen erfüllen, die aus einer Verwendung durch den Patienten selbst resultieren.

Aufgabe der vorliegenden Erfindung ist es daher, eine Vorrichtung der einleitend genannten Art derart zu konstruieren, daß eine vielseitige Einstellbarkeit bei gleichzeitig einfacher Bedienung bereit gestellt wird.

Diese Aufgabe wird erfindungsgemäß dadurch gelöst, daß das Stirnpolster als ein elastomeres Band ausgebildet ist, das von einer gabelartigen Halterung positioniert ist. Eine besonders gute Anpassung an eine Stirnkontur wird dadurch unterstützt.

Zur Fixierung einer Adaption an eine jeweilige Stirnhöhe wird vorgeschlagen, daß für die Verstelleinrichtung eine Arretierung in Richtung einer Längsachse des Halterungsschenkels vorgesehen ist.

Zur Fixierung einer Adaption an einen jeweiligen Stirnabstand wird vorgeschlagen, daß für das Stützelement eine Arretierung quer zur Längsachse vorgesehen ist.

Zur Erleichterung einer Einstellung ist vorgesehen, daß für die Führung der Verstelleinrichtung in Richtung der Längsachse eine Rastung vorgesehen ist.

Darüber hinaus ist auch daran gedacht, daß die Verstelleinrichtung in Richtung der Längsachse stufenlos verstellbar ist.

Eine weitere Bedienungserleichterung kann dadurch erreicht werden, daß für die Verstellung des Stützelementes quer zur Längsachse eine Rastung vorgesehen ist.

Auch im Hinblick auf diese Verstellmöglichkeit ist daran gedacht, daß das Stützelement quer zur Längsachse stufenlos verstellbar ist.

Ebenfalls ist es möglich, daß das Stirnpolster als ein Schaumpolster ausgebildet ist.

Eine weitere Erhöhung der Einstellungsflexibilität kann dadurch erreicht werden, daß das Stützelement relativ zum Halterungsschenkel verschwenkbar angeordnet ist.

Ebenfalls ist daran gedacht, daß der Halterungsschenkel relativ zu einem Maskengrundkörper der Atemmaske verschwenkbar angeordnet ist.

Eine weitere Anpassung an einen individuellen Patienten kann dadurch erreicht werden, daß das Stirnpolster in frontaler Richtung verkippbar angeordnet ist.

Ein weiter gesteigerter Benutzungskomfort kann dadurch erreicht werden, daß das Stirnpolster seitlich kippbar angeordnet ist.

Gemäß einer weiteren Ausführungsvariante ist daran gedacht, daß das elastomere Band einteilig mit der gabelartigen Halterung konstruiert ist.

Die Figuren 1 bis 22 zeigen insgesamt keine Ausführungsbeispiele der Erfindung, sondern es handelt sich um Beispiele, die das Verständnis der Erfindung erleichtern.

In den Zeichnungen 23 und 24 sind Ausführungsbeispiele eines erfindungsgemäßen Stirnpolsters schematisch dargestellt. Es zeigen:
- Fig. 1: Eine perspektivische Darstellung einer Atemmaske mit einem Stützelement, das sowohl in Richtung einer Längsachse eines Halterungsschenkels als auch quer zur Längsachse positionierbar ist,
- Fig. 2: eine Ansicht gemäß Blickrichtung II in Fig. 1,
- Fig. 3: eine Ansicht gemäß Blickrichtung III in Fig. 1,
- Fig. 4: eine perspektivische Darstellung gemäß Blickrichtung IV in Fig. 3,
- Fig. 5: eine Explosionsdarstellung des Stützelementes mit Stirnpolster und Verstelleinrichtung,
- Fig. 6: eine Darstellung der Anordnung gemäß Blickrichtung VI in Fig. 5,
- Fig. 7: eine vergrößerte perspektivische Darstellung des Stützelementes,
- Fig. 8: eine Seitenansicht des Stützelementes gemäß Blickrichtung VIII in Fig. 7,
- Fig. 9: eine vergrößerte perspektivische Darstellung des Stirnpolsters,
- Fig. 10: eine vergrößerte perspektivische Darstellung der Verstelleinrichtung,
- Fig. 11: eine Darstellung der Verstelleinrichtung gemäß Blickrichtung XI in Fig. 10,
- Fig. 12: die Verstellvorrichtung gemäß Blickrichtung XII in Fig. 10,
- Fig. 13: eine Seitenansicht gemäß Blickrichtung XIII in Fig. 1,
- Fig. 14: eine weitere perspektivische Darstellung der Verstelleinrichtung,
- Fig. 15: einen Längsschnitt gemäß Schnittlinie XV-XV in Fig. 11 nach einer Drehung um 90°,
- Fig. 16: eine perspektivische Darstellung der Verstelleinrichtung gemäß Blickrichtung XVI in Fig. 14,
- Fig. 17: eine weitere perspektivische Darstellung der Verstelleinrichtung,
- Fig. 18: eine weitere perspektivische Darstellung des Stützelementes,
- Fig. 19: eine weitere perspektivische Darstellung des Stirnpolsters,
- Fig. 20: eine perspektivische Darstellung gemäß Blickrichtung XX in Fig. 19 nach einer Drehung um 90°,
- Fig. 21: eine Ansicht gemäß Blickrichtung XXI in Fig. 19,
- Fig. 22: einen Querschnitt gemäß Schnittlinie XXII-XXII in Fig. 19,
- Fig. 23: eine Prinzipdarstellung einer brückenartigen Stirnstütze und
- Fig. 24: eine gegenüber Fig. 23 abgewandelte Konstruktion einer brückenförmigen Stirnstütze in einteiliger Ausführung.

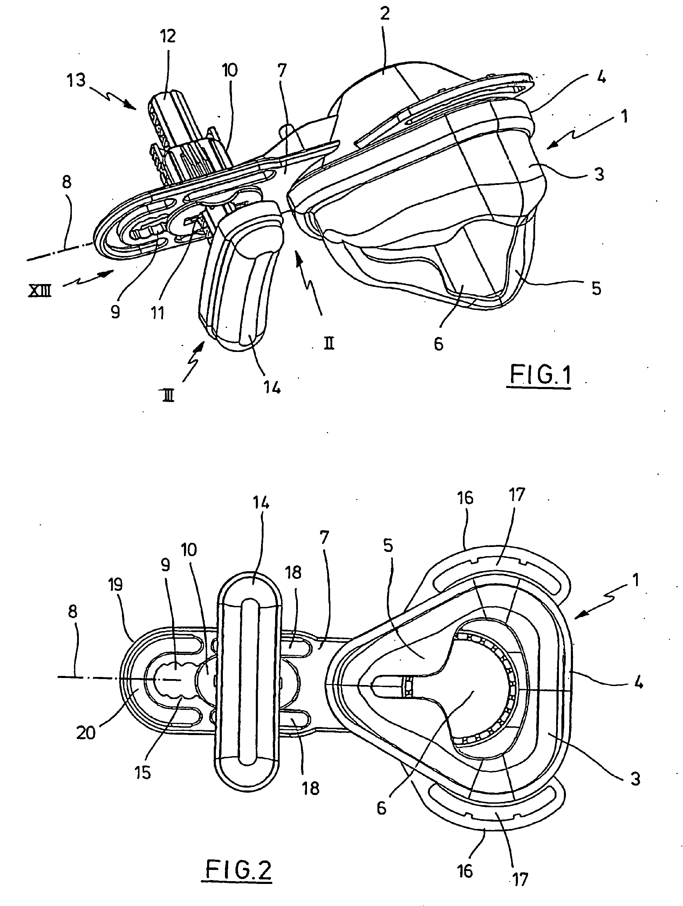

Fig. 1 zeigt eine perspektivische Darstellung einer Atemmaske (1), die im wesentlichen aus einem Maskengrundkörper (2) aus einem festen Material und einem Maskenpolster (3) aus einem weichen Material besteht. Das Maskenpolster (3) kann über einen Polstersockel (4) auf einen Rand des Maskengrundkörpers (2) aufgesteckt sein. Das Maskenpolster (3) begrenzt mit Dichtungslippen (5) eine Atemöffnung (6), in die bei einer Benutzung durch einen Patienten eine Nase des Patienten eingeführt wird.

Am Maskengrundkörper (2) ist ein Halterungsschenkel (7) befestigt, der sich mit einer Längsachse (8) im wesentlichen parallel zu einer von der Atemöffnung (6) aufgespannten Ebene erstreckt. Der Halterungsschenkel (7) weist eine Montageöffnung (9) auf, in die eine Verstelleinrichtung (10) eingesetzt ist. Die Verstelleinrichtung (10) weist quer zur Längsachse (8) eine Führungsausnehmung (11) auf, in die ein Schaft (12) eines Stützelementes (13) eingeführt ist. Das Stützelement (13) trägt ein Stirnpolster (14).

Aus der Darstellung in Fig. 2 ist erkennbar, daß sich das Stirnpolster (14) im wesentlichen quer zur Längsachse (8) erstreckt. Ebenfalls ist zu erkennen, daß die Montageöffnung (9) ähnlich zu einem Längsschlitz ausgebildet ist, der sich in Richtung der Längsachse (8) erstreckt. Gemäß dem Beispiel in Fig. 1 und Fig. 2 weist die Montageöffnung (9) mehrere Rastungen (15) auf, um eine stufenweise definierte Positionierung der Verstelleinrichtung (10) in Richtung der Längsachse (8) zu unterstützen. Fig. 2 veranschaulicht ebenfalls, daß am Maskengrundkörper (2) seitlich zwei Flansche (16) angeformt sind, die jeweils mit schlitzförmigen Ausnehmungen (17) versehen sind.

Alternativ zu einer Verwendung von Rastungen (15) ist es auch denkbar, für die Positionierung der Verstelleinrichtung (10) in Richtung der Längsachse (8) mehrere Aufnahmen im Bereich des Halterungsschenkels (7) anzuordnen und die Verstelleinrichtung (10) jeweils in eine dieser Aufnahmen einzusetzen.

Im Bereich des Halterungsschenkels (7) sind seitlich zwei Ausnehmungen (18) und im Bereich eines Endsegmentes (19) ist eine hufeisenartige Ausnehmung (20) angeordnet.

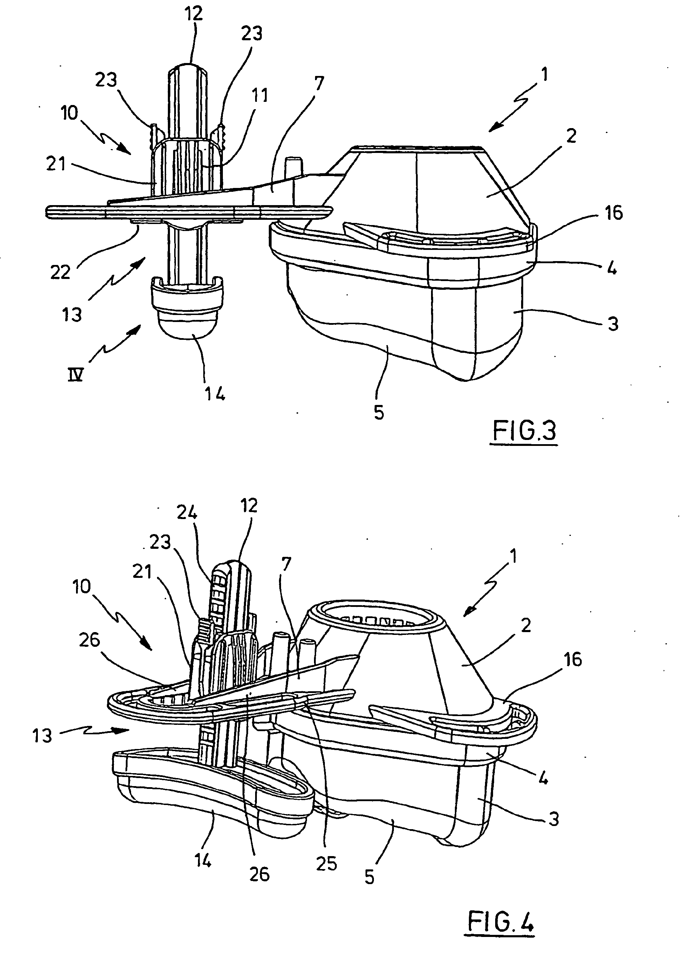

Aus Fig. 3 ist erkennbar, daß die Verstelleinrichtung (10) mit einem Grundkörper (21) durch die Montageöffnung (9) hindurchragt und mit einer Platte (22) auf dem Halterungsschenkel (7) aufliegt. Der Grundkörper (21) weist federnd gelagerte Klemmstege (23) auf, die den Schaft (12) des Stützelementes (13) innerhalb der Führungsausnehmung (11) der Verstelleinrichtung (10) fixieren.

Die perspektivische Darstellung in Fig. 4 veranschaulicht, daß der Schaft (12) im Bereich seiner den Klemmstegen (23) zugewandten Seiten mit einer Rastung (24) versehen ist, um auch hier eine definierte und schrittweise Positionierung des Stützelementes (13) zu ermöglichen.

Fig. 4 veranschaulicht ebenfalls, daß der Halterungsschenkel (7) im wesentlichen aus einer Schenkelplatte (25) sowie Stützstegen (26) besteht, die im wesentlichen senkrecht zur Schenkelplatte (25) angeordnet sind und zu einer erhöhten Biegesteifigkeit beitragen. Die Stützstege (26) verjüngen sich in eine dem Maskengrundkörper (2) abgewandte Richtung und sind im Bereich ihrer verdickten Enden am Maskengrundkörper (2) angeformt.

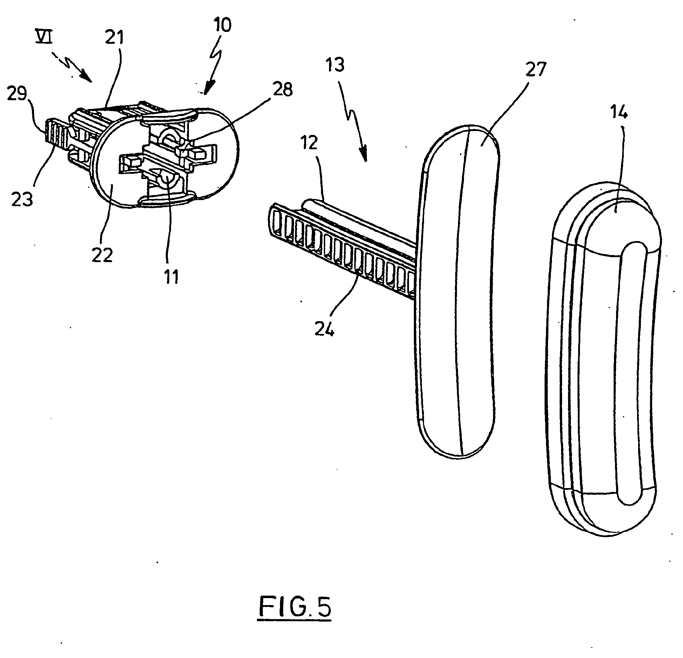

Fig. 5 veranschaulicht in einer Explosionsdarstellung die Konstruktion der Verstelleinrichtung (10) und des Stützelementes (13). Insbesondere ist erkennbar, daß das Stützelement (13) eine Stützplatte (27) aufweist, auf die das Stirnpolster (14) aufgesteckt wird. Ebenfalls ist aus Fig. 5 zu erkennen, daß die Klemmstege (23) mit Rastvorsprüngen (28) versehen sind, die in die Rastungen (24) eingreifen können. Bei einem manuellen Druck auf Bedienflächen (29) der Klemmstege (23) werden die Rastvorsprünge (28) aus der Rastung (24) herausgeführt.

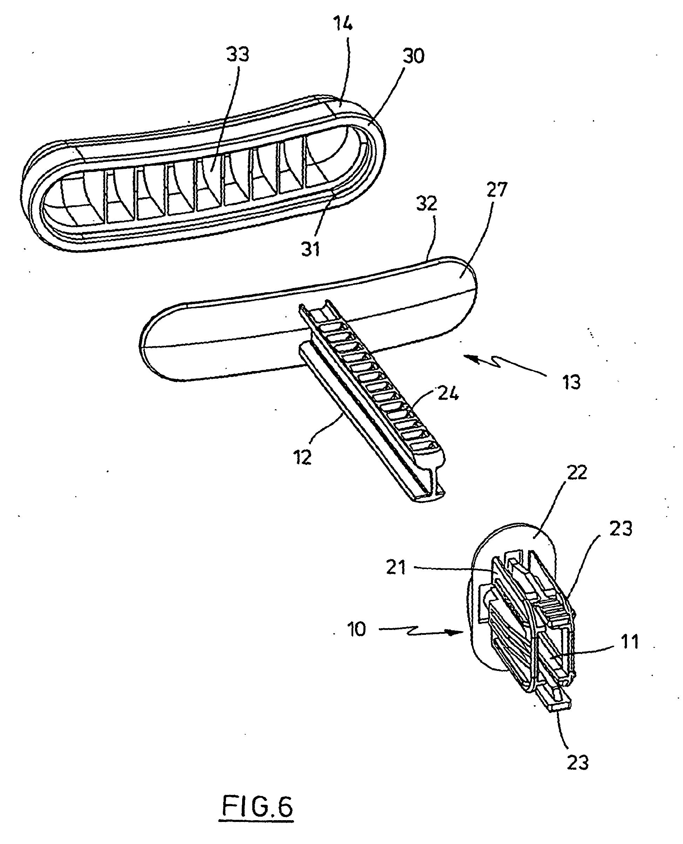

Fig. 6 veranschaulicht, daß das Stirnpolster (14) im Bereich eines Randes (30) mit einem U-Profil (31) versehen ist, das auf einen Rand (32) der Stützplatte (27) aufsteckbar ist. Darüber hinaus weist das Stirnpolster (14) zur Bereitstellung einer ausreichenden Eigenstabilität innere Querstege (33) auf, die bei einer Ausbildung des Stirnpolsters (14) aus einem elastomeren Material zu einem guten Kompromiß zwischen einer ausreichenden Formstabilität und einer ausreichenden Nachgiebigkeit beitragen.

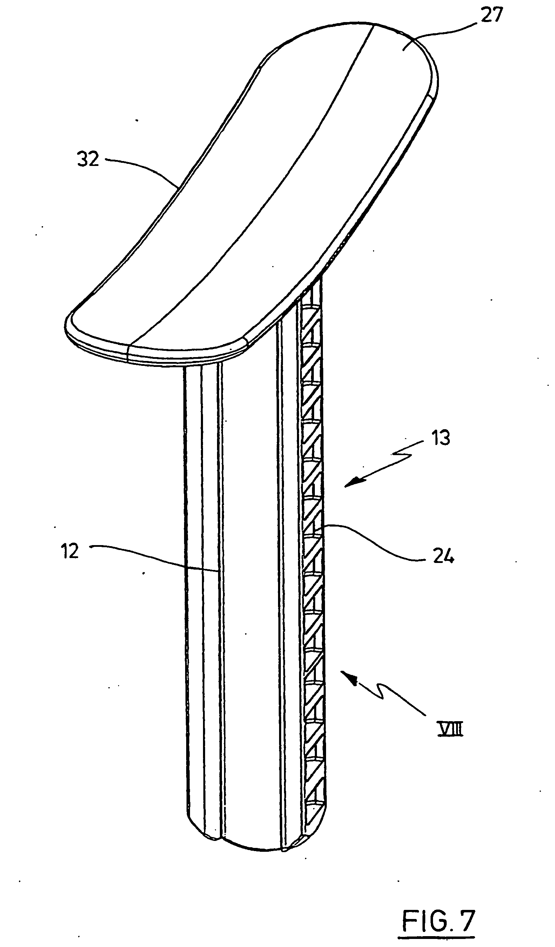

Die Darstellung des Stützelementes (13) in Fig. 7 veranschaulicht, daß durch die Rastung (24) eine Vielzahl von Rasterfächern ausgebildet werden, die jeweils zur Aufnahme der Rastvorsprünge (28) der Klemmstege (23) vorgesehen sind. Ebenfalls ist erkennbar, daß die Stützplatte (27) leicht gewölbt ist, um eine Anpassung an die Kontur einer menschlichen Stirn zu gewährleisten.

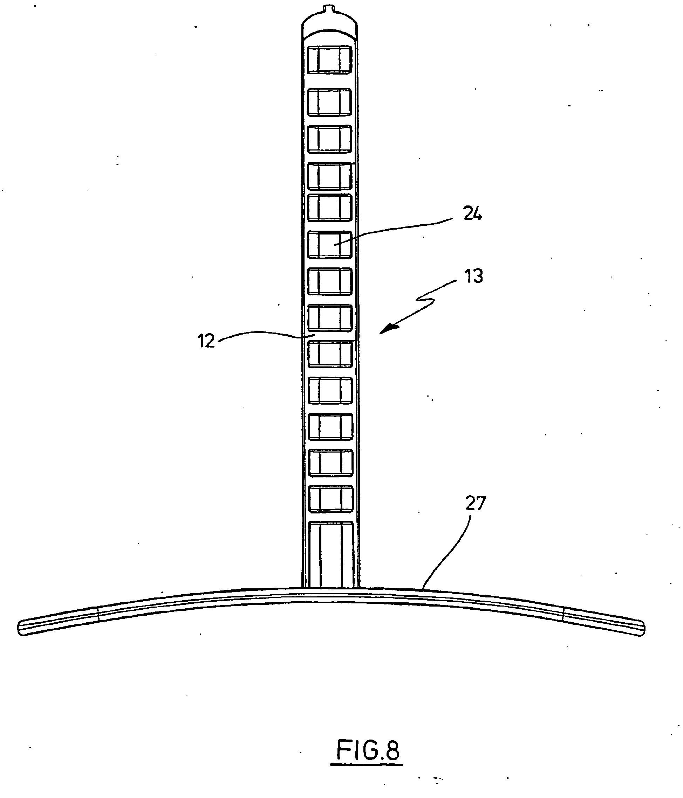

Die Wölbung der Stützplatte (27) sowie die Anordnung der Rastung (24) entlang der Längserstreckung des Schaftes (12) wird in Fig. 8 noch einmal veranschaulicht.

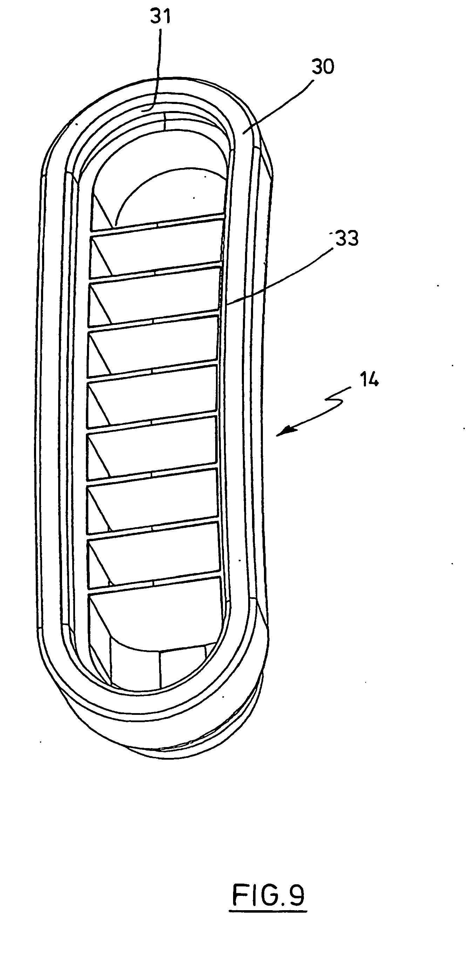

Aus der Darstellung in Fig. 9 ist der Aufbau des Stirnpolsters (14) in größerer Detailliertheit zu erkennen. Das Stirnpolster (14) weist eine haubenartige Querschnittgestaltung auf und von den Querstegen (33) werden sowohl Längsseiten des Stirnpolsters (14) relativ zueinander abgestützt als auch eine Versteifung im Bereich einer dem Rand (30) abgewandten Abschlußrundung hervorgerufen.

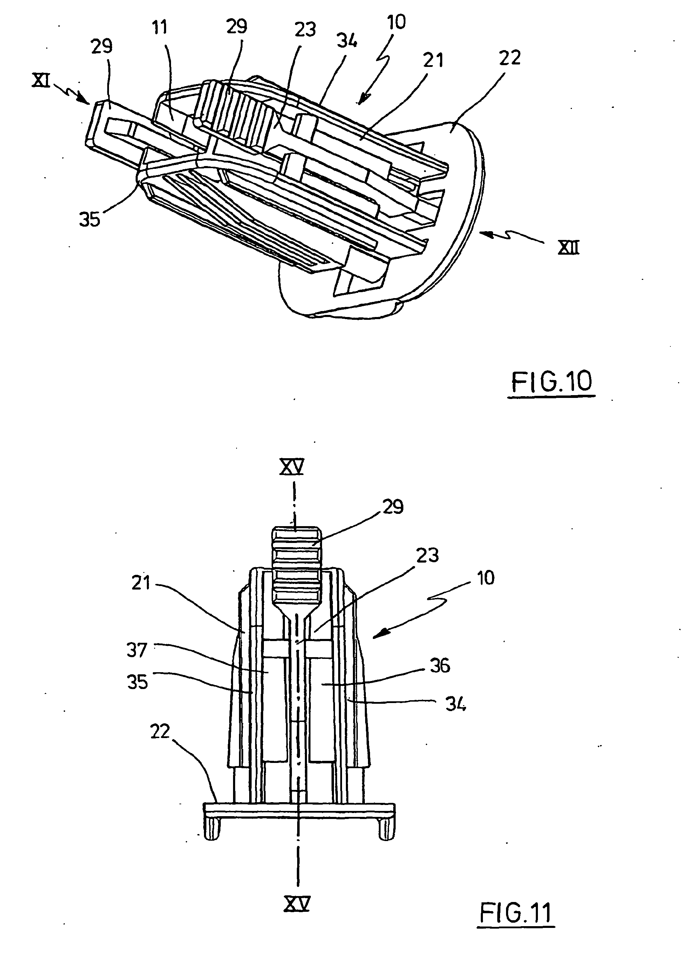

Die vergrößerte perspektivische Darstellung in Fig. 10 zeigt die Verstelleinrichtung (10) in stärkerer Detailliertheit. Es ist zu erkennen, daß der Grundkörper (21) im wesentlichen aus zwei Platten (34, 35) besteht, zwischen denen die Klemmstege (23) angeordnet sind.

Aus Fig. 11 ist zu erkennen, daß an den Platten (34, 35) Querstege (36, 37) angeordnet sind, die sich in Richtung auf die Klemmstege (23) erstrecken.

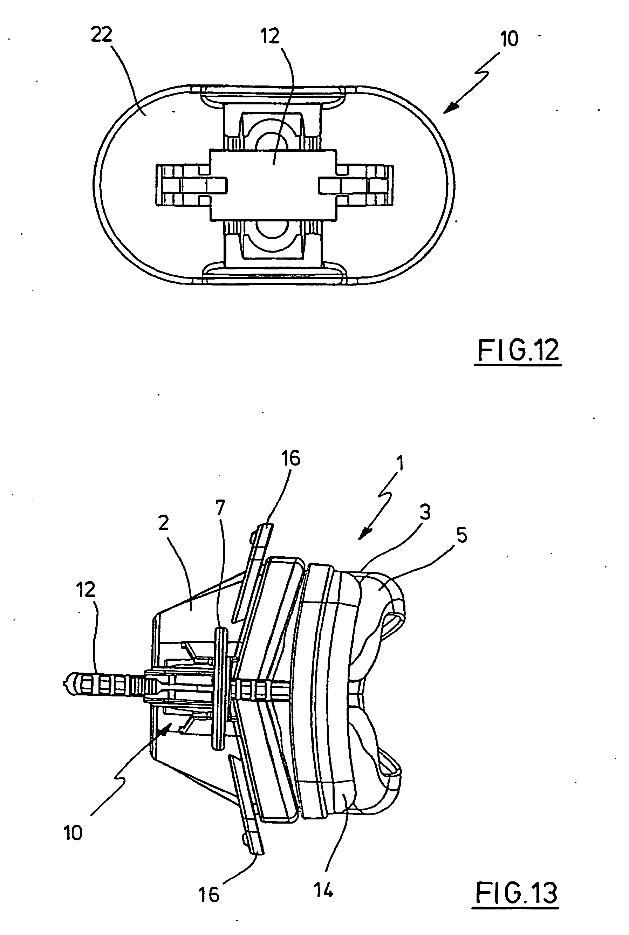

Fig. 12 veranschaulicht die Kombination der Verstelleinrichtung (10) mit dem Schaft (12). Fig. 13 zeigt in einer weiteren Seitenansicht als Zusammenbaudarstellung die Kombination der Atemmaske (1) mit einem Stirnpolster (14), das über einen Schaft (12) von einer Verstelleinrichtung (10) im Bereich des Halterungsschenkels (7) fixiert ist.

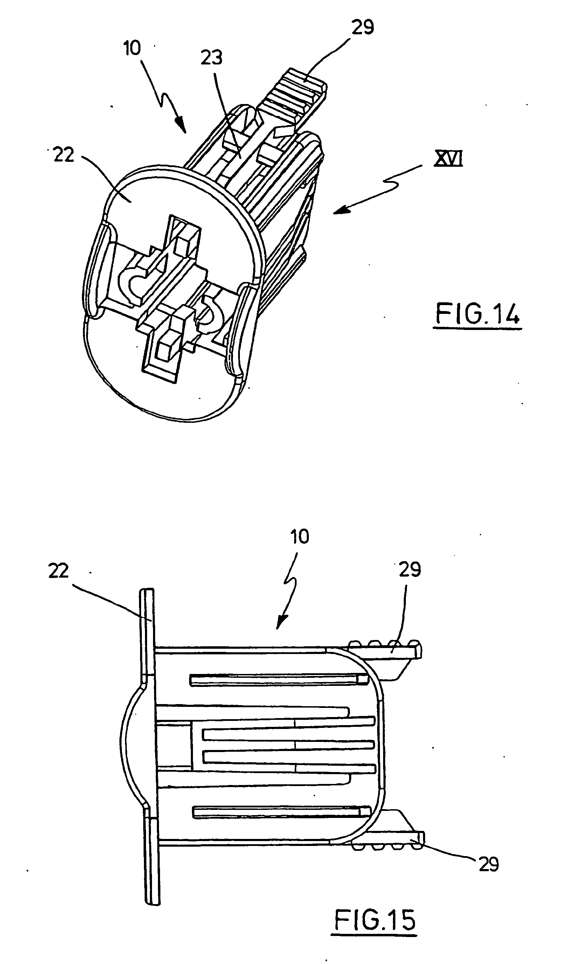

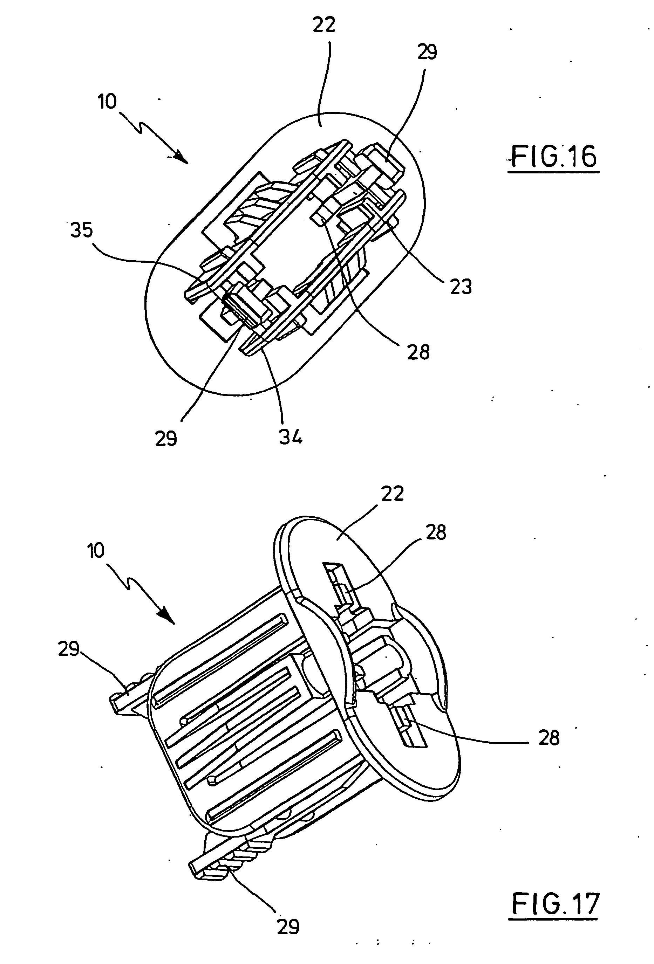

Fig. 14 und Fig. 15 zeigen nochmals den Aufbau der Verstelleinrichtung (10). Weitere Darstellungen der Verstelleinrichtung (10) finden sich auch in Fig. 16 und in Fig. 17.

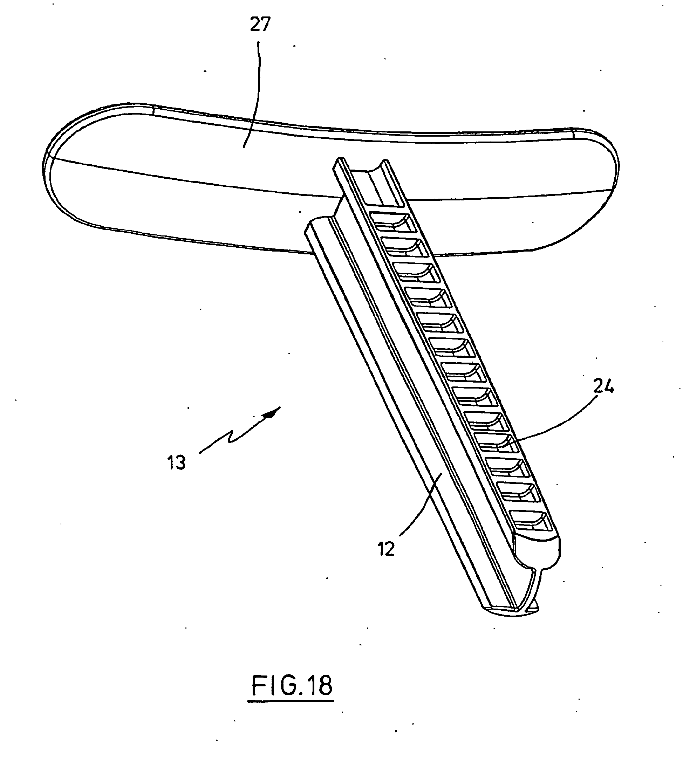

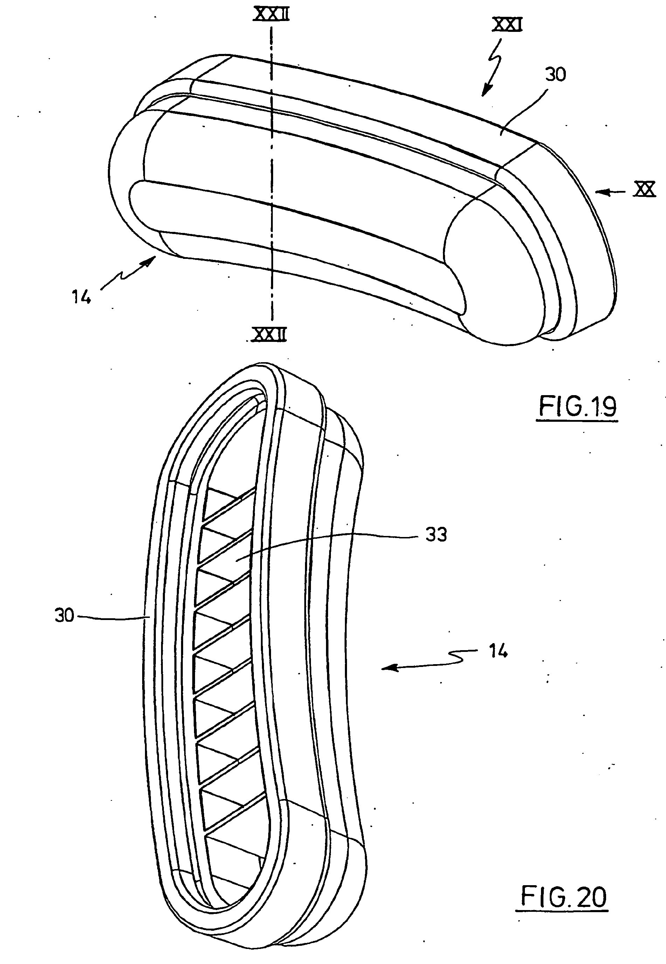

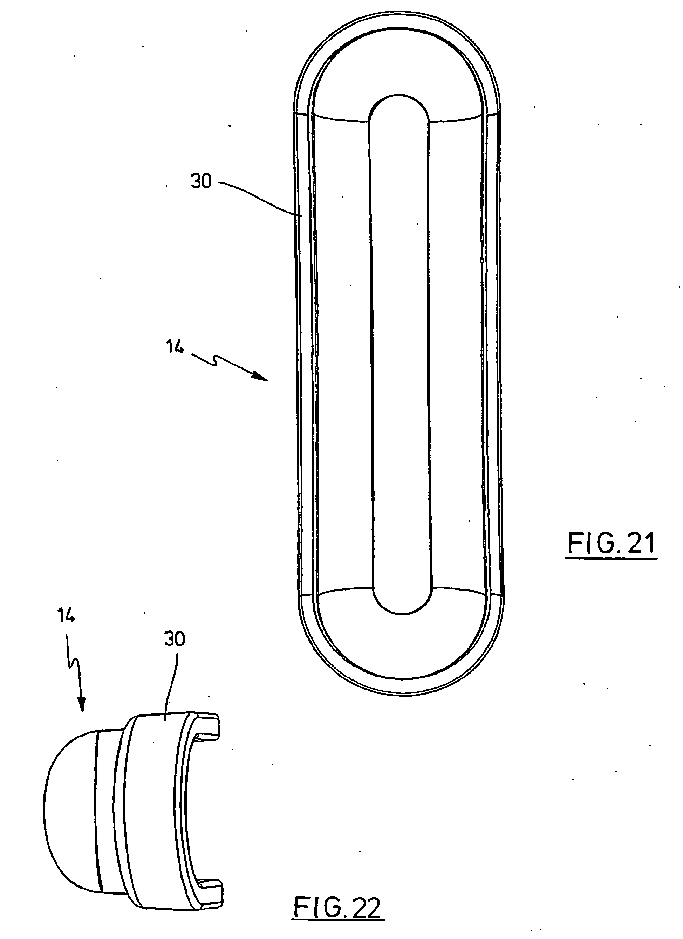

Fig. 18 zeigt in einer weiteren perspektivischen Darstellung nochmals das Stützelement (13), wobei insbesondere erkennbar ist, daß der Schaft (12) ein Querschnittprofil ähnlich zu einem I aufweist. Fig. 19 und Fig. 20 zeigen nochmals in weiteren perspektivischen Darstellungen die Gestaltung des Stirnpolsters (14).

Fig. 21 veranschaulicht, daß das Stirnpolster (14) im wesentlichen eine symmetrische Gestaltung zu seiner Längsachse aufweist. Fig. 22 veranschaulicht die gerundete äußere Konturierung des Stirnpolsters (14).

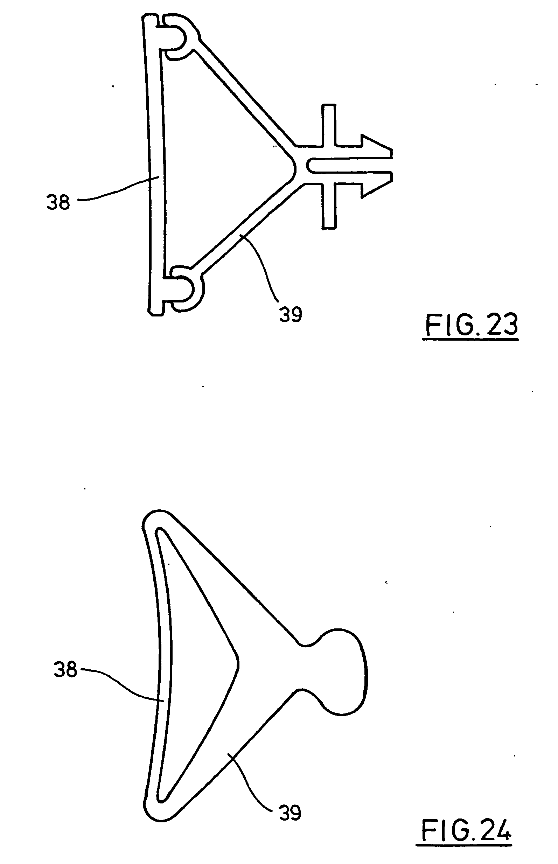

Fig. 23 zeigt eine Ausführungsform eines erfindungsgemäßen Stirnpolsters, bei der ein elastomeres Band (38) von einer gabelartigen Halterung (39) positioniert ist. Das elastomere Band (38) kann sich der Stirnkontur und der Stirnneigung anpassen.

Fig. 24 zeigt eine zur Fig. 23 modifizierte Ausführungsform, bei der das elastomere Band (38) und die Halterung (39) einteilig ausgeführt sind. Als Werkstoff kann z.B. Schaum oder ein Hohlkörper verwendet werden.

Ein typischer Einstellvorgang erfolgt derart, daß der Patient zunächst die Atemmaske (1) im Bereich seiner Nase positioniert und in einem ersten Schritt eine Anpassung an die Stirnhöhe durch eine Verschiebung der Verstelleinrichtung (10) innerhalb der Montageöffnung (9) in Richtung der Längsachse (8) durchführt. Über die Rastung (15) wird eine definierte Positionierung unterstützt. Nach einer geeigneten Anordnung und Arretierung der Verstelleinrichtung (10) innerhalb der Montageöffnung (9) wird eine Anpassung an den Stirnabstand durch ein Herausziehen des Schaftes (12) aus der Führungsausnehmung (11) bzw. durch ein Hineinschieben des Schaftes (12) in die Führungsausnehmung (11) durchgeführt. Vor dieser Positionierung werden durch einen Druck auf die Bedienflächen (29) der Klemmstege (23) die Rastvorsprünge (28) aus den Vertiefungen der Rastung (24) herausgeschwenkt. Nach einer geeigneten Positionierung des Schaf-tes (12) des Stützelementes (13) werden die Bedienflächen (29) losgelassen und die Rastvorsprünge (28) federn in die Rastung (24) zurück. Hierdurch ist das Stützelement (13) in seiner aktuellen Positionierung arretiert.

Sollte eine nachträgliche Veränderung der gewählten Positionierungen erforderlich sein, so kann sowohl eine Änderung der Stirnhöheneinstellung als auch eine Änderung der Stirnabstandseinstellung ohne Rückwirkung auf die jeweils andere Einstellung erfolgen. Die erläuterten Arretierungen vermeiden eine ungewollte Veränderung der Positionierung des Stützelementes (13) während der Benutzung.

## Patentansprüche

1. Atemmaske (1) mit einer Vorrichtung zur Abstützung der Atemmaske (1) im Bereich einer Stirn eines Patienten, die mindestens ein im Bereich mindestens eines Halterungsschenkels (7) der Atemmaske (1) angeordnetes und über eine verstelleinrichtung (10) mit dem Halterungsschenkel (7) verbundenes Stützelement (13) aufweist, wobei das Stützelement (13) ein Stirnpolster (14) trägt, **dadurch gekennzeichnet, daß** das Stirnpolster (14) als ein elastomeres Band (38) ausgebildet ist, das von einer gabelartigen Halterung (39) positioniert ist.

2. Atemmaske nach Anspruch 1, **dadurch gekennzeichnet, daß** für die Verstelleinrichtung (10) eine Arretierung in Richtung einer Längsachse (8) des Halterungsschenkels (7) vorgesehen ist.

3. Atemmaske nach Anspruch 2, **dadurch gekennzeichnet, daß** für das Stützelement (13) eine Arretierung quer zur Längsachse (8) vorgesehen ist.

4. Atemmaske nach einem der Ansprüche 2, bis 3, **dadurch gekennzeichnet, daß** für die Führung der Verstelleinrichtung (10) in Richtung der Längsachse (8) eine Rastung vorgesehen ist.

5. Atemmaske nach einem der Ansprüche 2 bis 3, **dadurch gekennzeichnet, daß** die Verstelleinrichtung (10) in Richtung der Längsachse (8) stufenlos verstellbar ist.

6. Atemmaske nach einem der Ansprüche 2 bis 5, **dadurch gekennzeichnet, daß** für die Verstellung des Stützelementes (13) quer zur Längsachse (8) eine Rastung vorgesehen ist.

7. Atemmaske nach einem der Ansprüche 2 bis 5, **dadurch gekennzeichnet, daß** das Stützelement (13) quer zur Längsachse (8) stufenlos verstellbar ist.

8. Atemmaske nach einem der Ansprüche 1 bis 7, **dadurch gekennzeichnet, daß** das Stirnpolster (14) als ein Schaumpolster ausgebildet ist.

9. Atemmaske nach einem der Ansprüche 1 bis 8, **dadurch gekennzeichnet, daß** das Stützelement (13) relativ zum Halterungsschenkel (7) verschwenkbar angeordnet ist.

10. Atemmaske nach einem der Ansprüche 1 bis 9, **dadurch gekennzeichnet, daß** der Halterungsschenkel (7) relativ zu einem Maskengrundkörper (2) der Atemmaske (1) verschwenkbar angeordnet ist.

11. Atemmaske nach einem der Ansprüche 1 bis 10, **dadurch gekennzeichnet, daß** das Stirnpolster (14) in frontaler Richtung verkippbar angeordnet ist.

12. Atemmaske nach einem der Ansprüche 1 bis 11, **dadurch gekennzeichnet, daß** das Stirnpolster (14) seitlich kippbar angeordnet ist.

13. Atemmaske nach einem der Ansprüche 1 bis 12, **dadurch gekennzeichnet, daß** das elastomere Band (38) einteilig mit der gabelartigen Halterung (39) konstruiert ist.

14. Atemmaske nach einem der Ansprüche 1 bis 13, **dadurch gekennzeichnet, daß** der Halterungsschenkel (7) relativ zu einem Maskengrundkörper (2) der Atemmaske (1) verschwenkbar angeordnet ist und daß das elastomere Band (38) von der gabelartigen Halterung (39) in mindestens zwei unterschiedlichen Positionierungen entlang einer Längsachse (8) des Halterungsschenkels (7) arretierbar ist.

## Claims

1. Respiratory mask (1) with a device for supporting the respiratory mask (1) in the region of a brow of a patient, with at least one support element (13) disposed in the region of at least one retaining leg (7) of the respiratory mask (1) and connected to the retaining leg (7) via an adjusting mechanism (10), which support element (13) bears a brow pad (14), **characterised in that** the brow pad (14) is provided in the form of an elastomeric strip (38) which is positioned by means of a fork-type holder (39).

2. Respiratory mask as claimed in claim 1, **characterised in that** a lock is provided for the adjusting mechanism (10) in the direction of a longitudinal axis (8) of the retaining leg (7).

3. Respiratory mask as claimed in claim 2, **characterised in that** a lock is provided for the support element (13) transversely to the longitudinal axis (8).

4. Respiratory mask as claimed in one of claims 2 to 3, **characterised in that** a latch is provided for guiding the adjusting mechanism (10) in the direction of the longitudinal axis (8).

5. Respiratory mask as claimed in one of claims 2 to 3, **characterised in that** the adjusting mechanism (10) can be infinitely adjusted in the direction of the longitudinal axis (8).

6. Respiratory mask as claimed in one of claims 2 to 5, **characterised in that** a latch is provided as a means of adjusting the support element (13) transversely to the longitudinal axis (8).

7. Respiratory mask as claimed in one of claims 2 to 5, **characterised in that** the support element (13) can be infinitely adjusted transversely to the longitudinal axis (8).

8. Respiratory mask as claimed in one of claims 1 to 7, **characterised in that** the brow pad (14) is provided in the form of a foam pad.

9. Respiratory mask as claimed in one of claims 1 to 8, **characterised in that** the support element (13) is disposed so that it can be pivoted relative to the retaining leg (7).

10. Respiratory mask as claimed in one of claims 1 to 9, **characterised in that** the retaining leg (7) is disposed so that it can be pivoted relative to a mask base body (2) of the respiratory mask (1).

11. Respiratory mask as claimed in one of claims 1 to 10, **characterised in that** the brow pad (14) is disposed so that it can be tilted in the frontal direction.

12. Respiratory mask as claimed in one of claims 1 to 11, **characterised in that** the brow pad (14) is disposed so that it can be tilted sideways.

13. Respiratory mask as claimed in one of claims 1 to 12, **characterised in that** the elastomeric strip (38) is of an integral construction with the fork-type holder (39).

14. Respiratory mask as claimed in one of claims 1 to 13, **characterised in that** the retaining leg (7) is disposed so that it can be pivoted relative to a mask base body (2) of the respiratory mask (1) and the elastomeric strip (38) can be locked by the fork-type holder (39) in at least two different positions along a longitudinal axis (8) of the retaining leg (7).

## Revendications

1. Masque de respiration (1) avec un dispositif pour soutenir le masque de respiration (1) au niveau du front d'un patient, qui présente au moins un élément de soutien agencé au niveau d'au moins une poignée de support (7) du masque de respiration (1) et relié par un dispositif de réglage (10) à la poignée de support (7), où l'élément de soutien (13) porte un bourrelet frontal (14), **caractérisé en ce que** le bourrelet frontal (14) est formé par une bande élastomère (38), qui est positionnée par un soutien (39) de type fourche.

2. Masque de respiration selon la revendication 1, **caractérisé en ce qu'**un arrêt en direction d'un axe longitudinal (8) de la poignée de support (7) est prévu pour le dispositif de réglage (10).

3. Masque de respiration selon la revendication 2, **caractérisé en ce qu'**un arrêt transversal à l'axe longitudinal (8) est prévu pour l'élément de soutien (13).

4. Masque de respiration selon l'une des revendications 2 à 3, **caractérisé en ce qu'**une position de crantage est prévue pour le guidage du dispositif de réglage (10) en direction de l'axe longitudinal (8).

5. Masque de respiration selon l'une des revendications 2 à 3, **caractérisé en ce que** le dispositif de réglage (10) peut être réglé de manière continue en direction de l'axe longitudinal (8).

6. Masque de respiration selon l'une des revendications 2 à 5, **caractérisé en ce qu'**une position de crantage est prévue pour le réglage de l'élément de soutien (13), de manière transversale à l'axe longitudinal (8).

7. Masque de respiration selon l'une des revendications 2 à 5, **caractérisé en ce que** l'élément de soutien (13) peut être réglé de manière continue transversalement à l'axe longitudinal (8).

8. Masque de respiration selon l'une des revendications 1 à 7, **caractérisé en ce que** le bourrelet frontal (14) est formé comme un bourrelet en mousse.

9. Masque de respiration selon l'une des revendications 1 à 8, **caractérisé en ce que** l'élément de soutien (13) est agencé de manière mobile par rapport à la poignée de support (7).

10. Masque de respiration selon l'une des revendications 1 à 9, **caractérisé en ce que** l'élément de soutien (13) est agencé de manière mobile par rapport à un corps de base du masque (2) du masque de respiration (1).

11. Masque de respiration selon l'une des revendications 1 à 10, **caractérisé en ce que** le bourrelet frontal (14) est agencé de manière à pouvoir balancer, en direction frontale.

12. Masque de respiration selon l'une des revendications 1 à 11, **caractérisé en ce que** le bourrelet frontal (14) est agencé de manière à pouvoir balancer, de manière latérale.

13. Masque de respiration selon l'une des revendications 1 à 12, **caractérisé en ce que** la bande élastomère (38) est construite d'une pièce avec le support (39) de type fourche.

14. Masque de respiration selon l'une des revendications 1 à 13, **caractérisé en ce que** la poignée de support (7) est agencée de manière mobile par rapport à un corps de base de masque (2) du masque de respiration (1) et **en ce que** la bande élastomère (38) du support (39) de type fourche peut être arrêtée en au moins deux positions le long d'un axe longitudinal (8) de la poignée de support (7).
